# EUROPEAN PATENT SPECIFICATION

(11) **EP 0 562 720 B1**
(45) Date of publication and mention of the grant of the patent: **12.08.1998**
(21) Application number: 93301398.9
(22) Date of filing: 25.02.1993
(51) Int. Cl.: A61K 7/08

(54) **Hair shampoo**
Haarshampoo
Composition de shampooing pour cheveux

(30) Priority: 27.02.1992 GB 9204175
(43) Date of publication of application: 29.09.1993
(73) Proprietor: UNILEVER PLC, London EC4P 4BQ (GB); UNILEVER N.V., 3013 AL Rotterdam (NL)
(72) Inventor: Pratley, Stuart Keith, Unilever Research, Wirral, Merseyside L63 3JW (GB); Steer,David Carles, Unilever Research, Wirral, Merseyside L63 3JW (GB)
(74) Representative: Bristow, Stephen Robert

(56) References cited:
- EP-A- 0 398 177
- WO-A-92/08439
- WO-A-93/02657

## Description

### Field of the Invention

The invention relates to hair shampoos. In particular, the invention is concerned with shampoo compositions which contain alkyl polyglycosides.

### Background to the Invention and Prior Art

The most widely used surfactants in hair shampoos are alkyl sulphates, polyoxyethylene alkyl sulphates and alkyl benzene sulphonates. These compounds are known to have a good foaming and deterging power. Due to their harshness, however, they are not desirable as components for cleansing compositions topically applied to human skin and hair. Their damaging effect, particularly where young, tender or damaged skin is involved, has been the subject of intense study for many years.

Moreover, these widely used surfactants are not regarded as environmentally-friendly surfactants because their manufacture requires the use of large amounts of sulphuric acid; and in the case of the ethoxylated surfactants large quantities of ethylene oxide. These two starting materials are not only noxious but also very harmful to animals and plants.

Another group of conventionally used surfactants are phosphate surfactants. They are known to be very mild and to have a good foaming power. However, they are not environmentally-acceptable as biodegradation leads to phosphates which are very undesirable because of their damaging effect as water-borne nutrient.

There are several types of surfactants available which are regarded as environmentally-acceptable substances. Alkyl polyglycosides are one such group of surfactants. However, it has been observed that these surfactants are inferior in terms of their performance, such as foam quality. Consequently, there is a strong need for shampoos which, although containing environmentally-friendly surfactants, do not suffer from such draw-backs.

Another group of environmentally-friendly surfactant are the acyl lactylates. Their use in several types of cleansing products is known. Examples are given in the following patent documents.

US-A-3,728,447 (C J Patterson) discloses hair shampoo compositions containing fatty acid lactylates or glycolates. While the cleaning action of shampoos based on the fatty acid lactylates is satisfactory the foam is minimal. In order to achieve higher foaming action it is described to include harsh and environmentally-unfriendly detergents such as sodium lauryl sulphate or triethanolamine lauryl sulphate.

EP-A-278 370 describes shampoo compositions comprising a combination of 0.1-5% by weight of a fatty acid-lactic acid ester having a branched acyl group and 5-50% by weight of an anionic surfactant, such as salts of alkyl benzene sulphonic acids and salts of alkyl phosphates. The ratio of anionic surfactant to lactylate used in the examples lies in the range 2000:1 to 7.5:1, with 10:1 being most used.

EP-A-224 796 (Kao) describes a detergent composition comprising (a) a phosphate surfactant and (b) an acyl lactylate having an acyl group containing 12 to 18 carbon atoms. The detergent composition is said to have excellent foaming characteristics as well as excellent detergency and mildness to the skin and hair.

US-A-4,946,832 (RITA Corporation) describes cosmetic base compositions comprising 1 to 15% by weight sucrose fatty acid ester, 3 to 45% by weight acyl lactylate or its alkali metal salts and solvent. The compositions promote wound healing and reduce skin dryness.

A known problem associated with shampoo compositions formulated with natural surfactants such as alkyl polyglycosides is that the foam produced is harsh and can be thought of as unpleasant by the person using the shampoo. Applicants have unexpectedly discovered that a combination of acyl lactylates with alkyl polyglycosides provides an improved feel to the foam. Although containing mainly environmentally safe surfactants, the compositions so obtained are capable of producing a good lather and accordingly have great consumer appeal. Also the compositions are mild.

### Definition of the Invention

Accordingly, the invention provides a hair shampoo composition which comprises, in addition to water.
(a) one or more alkyl poly glycoside(s) where the alkyl group contains 5 to 30 carbon atoms;
(b) one or more acyl lactylate(s) of the following structure (1)

   (R¹CO-(O-CHCH₃-CO)ₐ O)_{b} M (1)

   where R¹CO represents a C₆ to C₃₄ acyl radical, preferably C₈ to C₁₈; a is an integer of from 1 to 4; b is 1 or 2; and M represents H or a cosmetically-acceptable counterion of the valency 1 or 2; and
   wherein at least one part of acyl lactylate is used for six parts of alkyl polyglycoside.

Preferably the ratio of (b) to (a) lies in the range 1:6 to 2:1.

### The alkyl polyglycoside

The hair shampoo composition according to the invention comprises one or more alkyl polyglycoside(s) where the alkyl group contains 5 to 30 carbon atoms.

Alkyl polyglycosides (APGs) are known in hair care and washing compositions. They have the advantage that they are very mild and readily biodegradable. Additionally, they are obtainable by reaction of glucose and fatty alcohols, both of which being derived from renewable resources, namely corn starch and vegetable oils, respectively. Consequently, APGs are regarded as environmentally-friendly substances.

Preferred APGs are represented by the following structure (2):

RO-(G)ₙ (2)

where R is a branched or straight-chain C₅ to C₃₀ saturated or unsaturated alkyl group, G is a saccharide group and n is an integer indicating the degree of polymerisation.

More preferably, R represents a C₈ to C₁₆, alkyl group and most preferably R represents a C₈ to C₁₄ alkyl group. Preferred mixtures of APGs can be described as those having a mean carbon number in the alkyl chain of from C₉ to C₁₁, based on % by weight of the APG.

G is preferably selected from C₅ or C₆ monosaccharide residues, such as residues of glucose, xylose, lactose, fructose, mannose and derivatives thereof. Most preferably G is a glucose residue.

The degree of polymerisation, n, is preferably in the range of from 1 to 10. If mixtures of APG's are used, then the mean degree of polymerisation is preferably in the range of about 1.1 to about 2.0 and more preferably about 1.3 to about 1.5, based on % by weight of the APG.

Suitable alkyl polyglycosides include the following commercially available materials:
Oramix NS10, ex Seppic, which is a mixture of alkyl polyglucosides having a mean carbon number of 10.4 in the alkyl group.
Plantaren 2000, which is a mixture of alkyl polyglucosides having a mean carbon numbers of 10.2 in the alkyl group and hence referred to as a decyl glucoside.
Plantaren 1200, which is a mixture of C₁₂, C₁₄ and C₁₆ alkyl polyclucosides.

The degree of polymerisation for all materials is about 1.4.

The amount of the alkyl polyglycoside(s) present in the cleansing composition according to the invention is preferably from 5 to 25% by weight and more preferably from 8 to 16% by weight of the composition.

### The acyl lactylate

The composition according to the invention comprises one or more acyl lactylate(s) of the following structure (1)

(R¹CO-(O-CHCH₃-CO)ₐ O)_{b} M (1)

where R¹CO represents a C₆ to C₃₄ acyl radical; a is an integer of from 1 to 4; b is 1 or 2; and M represents H or a cosmetically-acceptable counterion of the valency 1 or 2.

Preferably, R¹CO represents a C₈ to C₁₈ and more preferably a C₁₀ to C₁₄ acyl radical.

The preferred cosmetically acceptable counterion is chosen from hydrogen, alkali metal cations, alkaline earth metal cations, ammonium or a substituted ammonium ion having one or more C₁ to C₃ alkyl or hydroxy alkyl group(s). Hydrogen is the least preferred counterion. The value of b is selected such that the molecule of the acyl lactylate is electrically neutral. Thus, if the counterion has a valency of 2 then b is also 2.

Examples of acyl lactylates having the above structure include:
Sodium lauroyl monolactylate
Sodium myristoyl monolactylate
Sodium decanoyl monolactylate
Potassium lauroyl monolactylate
Potassium lauroyl dilactylate
Sodium myristoyl dilactylate
Sodium lauroyl dilactylate
Lauroyl dilactylic acid
Palmitoyl dilactylic acid
Triethanolammonium lauroyl monolactylate
Ammonium decanoyl monolactylate, and
Triethanolammonium decanoyl monolactylate.

It is also possible to use mixtures of the acyl lactylates. Examples for suitable mixtures are commercially available under the trade names Pationic 122A, which is a 1:1 mixture of sodium capryl/sodium lauroyl lactylate, and Pationic 138C, which is a 7:3 mixture of sodium lauroyl/sodium myristoyl lactylate.

The amount of acyl lactylate(s) present in the compositions according to the invention is preferably 3 to 15% by weight and more preferably 4 to 8% by weight of the composition.

One advantage of the acyl lactylates over sulphur or phosphorous - containing surfactants is that they are also free from any environmental concern. The starting materials used to produce acyl lactylates are environmentally-friendly materials, namely lactic acid and fatty acids. Both starting materials are derived from renewable resources with the lactic acid being produced by fermentation of glucose and the fatty acids being derived from vegetable oils. Additionally, biodegradation of the acyl lactylates results in the starting materials used for their manufacture and hence the acyl lactylates are advantageous in terms of the acceptability of their degradation products.

As acyl lactylates having a straight-chain acyl group are more readily bio-degradable than those having a branched acyl group, the straight chains are preferred.

### Combination of Acyl lactylate and APG

It is preferred that the amounts of acyl lactylate(s) and alkyl polyglycoside(s) are chosen such that the weight ratio of APG to acyl lactylate is less than or equal to 6:1. Preferably the ratio lies in the range of from about 6:1 to 1:2, and more preferably from about 3:1 to about 1:1.

The total amount of surfactant present in the hair shampoo composition according to the invention is preferably in the range of from 12 to 50% by weight. The hair shampoo composition according to the invention can be provided in the standard form containing a total surfactant amount of 12 to 24% by weight or in the form of concentrates usually comprising 25 to 50% by weight of surfactant, based on the total composition.

### Water

If no other components apart from the acyl lactylate(s) and alkyl polyglycoside(s) are present in the hair shampoo composition, then water will form the balance. It is usually present in amounts of from 40 to 80% by weight of the composition.

### Optional ingredients

The hair shampoo composition can also comprise optional ingredients to modify the physical or chemical characteristics of the composition, eg. product form, foaming properties, pH-value or shelf life.

Examples of ingredients which can be included are:

Co-surfactants, such as N-methyl-N-acyl taurates, acyl isethionates, alkyl esters of Ω-sulphonated carboxylic acids, acyl amido polyoxyethylene sulphates, acyl polyglyceride sulphates, mono alkyl sulphosuccinates, mono alkyl phosphates, alkyl polyoxyethylene acetate, N-acyl glutamates, N-acyl sarcosinates, N-acyl alaninates, N-acyl aspartates, poly(oxyalkylene) fatty alkyl ethers, N-substituted betaines, sultaines and alkyl amphocarboxylates. Especially preferred are those co-surfactants which can be regarded as environmentally-friendly.

Hair conditioning agents, such as silicone oils and quaternary derivatives of hydroxy propyl guar gum, the latter being commercially available under the trade name Jaguar.

Viscosity modifiers, such as hydroxyethyl cellulose.

Anti-dandruff agents, such as octipirox and zinc pyridine-ethione, also referred to as ZnPTO.

Preservatives, such as ethanol, benzoic acid, sodium benzoate, sorbic acid, alkali metal halides.

Agents for controlling pH, such as sodium hydroxide, citric acid, triethanolamine, potassium hydroxide, amino sorbitol. The pH controlling agents are preferably present in an amount sufficient to adjust the composition to a pH value in the range of 6 to 8.

Foam modifying agents, such as cationic polymers, especially quaternised ammonium hydroxy ethyl cellulose polymers, eg. available as polyquaternium-24 or polyquaternium-10.

### Form of the Composition

The hair shampoo composition can take the form of a liquid or gel, intended to be dispensed from a capped container such as a bottle or from a sachet or tube.

### Testing foam quality

The quality of the foam was assessed by trained panellists. The training programme was also used to select those panellists that could give a reasonable level of discrimination for the two attributes necessary for comparisons; ie. creaminess of foam and coarse/dry feel of the foam.

Two test solutions are used as 'standards'. One is a 16% solution of Oramix NS10 an APG ex Seppic and the other is a 14% solution of Sodium Lauryl ether sulphate.

Each panellist is given two 8g/8 inch hair switches. These are wetted with warm water prior to application of 1 ml of one of the surfactant solutions to each switch. Taking one switch at a time, the panellist generates lather by rubbing the switch between the hands. The quality of the foam from the two surfactant solutions is then assessed.

Creaminess is assessed from feeling the foam gently to assess the thickness/richness/density of the foam. Coarse/dry feel is assessed by rubbing some of the foam between the fingers to gauge friction. A coarse/dry foam gives more feeling of friction.

### Example 1 - Hair Shampoo

| | wt % |
|---|---|
| Decyl polyglucoside (Oramix NS10) | 10.00 |
| Pationic 122A | 6.00 |
| Ethylene glycol distearate (solids content 44%) | 6.00 |
| Guar hydroxy propyl trimethyl ammonium chloride (Jaguar C-13-S) | 0.30 |
| Silicone emulsion (BY 22 026; Dow Corning) | 1.20 |
| NaCl | 2.00 |
| Potassium hydroxide solution | to pH 6.5 - 7.0 |
| Perfume | 0.50 |
| Water | to 100.00 |
| Ratio of APG:lactylate = 5:3 | |

### Example 2 - Hair Shampoo

| | wt % |
|---|---|
| Decyl polyglucoside (Plantaren 2000) | 12.00 |
| Pationic 138C | 4.00 |
| Xanthan gum polysaccaride thickener | 0.30 |
| Potassium hydroxide solution | to pH 6.5 - 7.0 |
| Perfume | 0.50 |
| Water | to 100.00 |
| Ratio APG:lactylate = 3:1 | |

### Example 3 - Hair Shampoo

| | wt % |
|---|---|
| Decyl polyglucoside (Plantaren 2000) | 6.00 |
| Lauryl polyglucoside (Plantaren 1200) | 5.00 |
| Pationic 122A | 5.00 |
| Ethylene glycol distearate (Pearliser) | 6.00 |
| NaCl | 3.00 |
| Potassium hydroxide solution | to pH 6.5 - 7.0 |
| Perfume | 0.5 |
| Water | to 100.00 |
| Ratio APG:lactylate = 11:5 | |

### Example 4 - Hair Shampoo

| | wt % |
|---|---|
| Decyl polyglucoside (Plantaren 2000) | 24.00 |
| Pationic 138C | 8.00 |
| NaCl | 1.80 |
| Potassium hydroxide solution | to pH 6.5 - 7.0 |
| Perfume | 0.80 |
| Water | to 100.00 |
| Ratio APG:lactylate = 3:1 | |

### Example 5 - Shampoo

| | wt % |
|---|---|
| Potassium decanoyl monolactylate | 10.00 |
| Potassium decanoyl dilactylate | 10.00 |
| Decyl polyglucoside (n=1.44) | 5.00 |
| Glycerol | 5.00 |
| Trisodium citrate dihydrate (Thickener) | 1.50 |
| Sodium carboxymethyl cellulose (Thickener) | 1.00 |
| Citric acid | to pH 7.0-7.5 |
| Distilled water | to 100.00 |
| Ratio APG:lactylate = 1:4 | |

### Example 6

The objective of this test was to investigate the influence of addition of lactylate on lather quality from APG.

Trained panellists washed their own hair using a series of active solutions with different ratios of APG:Lactylate (constant 16% total active detergent). The active solution was dosed onto the head from a syringe (5.5 ml) by a supervisor. During the wash/rinse procedure the panellists were asked to score the products against various attributes by magnitude estimation. These scores were recorded by the supervisor.

| **SOLUTIONS** | | | | | | |
|---|---|---|---|---|---|---|
| | A | B | C | D | E | F |
| APG Plantaren 2000 (Decyl glucoside) | 16 | 14 | 12 | 10 | 8 | 12 |
| Pationic 138C (C12 Lactylate) | - | 2 | 4 | 6 | 8 | - |
| Pationic 122A (C10 Lactylate) | - | - | - | - | - | 4 |
| All solutions were adjusted to pH 7. | | | | | | |

### Results

Two attributes were found to be significantly improved by the addition of acyl lactylate. They were creaminess of lather and the reduction of the coarse/dry feel of the lather.

| Attribute | Rank Order (score) | Least sig. diff. |
|---|---|---|
| Creaminess | E(63) D(62) F(59) C(54) B(53) A(49) | 11.3 |
| Coarse/dry feel | E(26) D(28) C(32) F(42) A(49) B(55) | 15.7 |
| Note: For coarse/dry feel of foam, a low score is the better result. | | |

### Discussion

For these attributes, which relate to lather quality, the two products with highest ratio of lactylate (E and D) were significantly preferred to the control without lactylate (A). Product C (12/4 ratio APG/lactylate) was also significantly preferred on coarse/dry feel but not on creaminess. This suggests that a lactylate to APG ratio of more than 1:7 is required to get the required benefits.

The "coarse/dry feel" description was adopted as a result of a language generation session in which it was found to be the least ambiguous description for the sensory foam quality from APG (as compared to conventional shampoo actives).

### Example 7

The objective of this test was to investigate the influence of lactylate on lather quality from nonionic actives, including APGs.

Panellists were trained to distinguish between lather quality and different active types. Descriptors were generated during a language generation session in which foams from different active types were assessed.

A panellist is given an 8g (8inch) hair switch which is then wetted in warm tap water. The test supervisor doses 0.8g of the active solution onto the switch which the panellists then rubs between her or his hands to generate foam. During this foam generating process and during the following rinse the panellist is asked to score the product against various attributes by magnitude estimation.

| **SOLUTIONS** | | | | | |
|---|---|---|---|---|---|
| | (%w/w active ingredient) | | | | |
| | A | B | C | D | E |
| Plantaren 2000 | 16 | 10 | 14.7 | - | - |
| Pationic 138C | - | 6 | 1.3 | - | 6 |
| Dobanol 91-6 | - | - | - | 16 | 10 |
| Water | to 100 | | | | |
| The pH of all solutions was adjusted to 6.5 - 7.5. Dobanol 91-6 is C9-11 6EO alcohol ethoxylate NB - Solutions A and C are outside the scope of the present invention and are included for comparative purposes only. | | | | | |

### Results

### Test 1

| Attribute | Rank Order (score) | Least sig. diff. |
|---|---|---|
| Creaminess of foam | B(63) C(41) A(34) | 12.1 |
| Coarse/dry feel of foam | B(21) C(51) A(63) | 19.7 |

### Test 2

| Attribute | Rank Order (score) | Least sig. diff. |
|---|---|---|
| Creaminess | B(66) E(33) D(28) | 14.5 |
| Coarse/dry | B(20) E(32) D(68) | 19.1 |

At the low level of lactylate used in EP 278370 (solution C, Test 1)) the inclusion of lactylate did not give a significant benefit. This is consistent with the results from the salon test described in Example 6. It can be concluded that the ratio of lactylate to APG, should be greater than 1:7; that is 1:6 or better for significant benefit to be obtained.

Test 2 shows that the enhanced foam properties obtained by using acyl lactylates with APGs are significantly more pronounced than that change in properties when the acyl lactylates are used with another nonionic surfactant, such as Dobanol 91-6.

## Claims

1. A hair shampoo composition which comprises, in addition to water,
(a) one or more alkyl polyglycoside(s) where the alkyl group contains 5 to 30 carbon atoms
(b) one or more acyl lactylate(s) of the following structure (1)
(R¹CO-(O-CHCH₃-CO)ₐ O)_{b} M
where R¹CO represents a straight chain or branched C₆ to C₃₄ acyl radical: a is an integer of from 1 to 4; b is 1 or 2; and M represents H or a cosmetically-acceptable counterion of the valency 1 or 2;
wherein the weight ratio of acyl lactylate(s) to alkyl polyglycoside(s) is greater than or equal to 1:6.

2. A composition according to claim 1 wherein the weight ratio of acyl lactylate(s) to alkyl polyglycoside(s) is in the range of from about 1:6 to 2:1.

3. A hair shampoo composition according to claim 1 or claim 2, wherein R¹CO represents a C₈ to C₁₈ straight chain acyl radical.

4. A hair shampoo composition according to any preceding claim, wherein R¹CO represents a C₁₀ to C₁₄ straight chain acyl radical.

5. A hair shampoo composition according to any preceding claim, wherein the alkyl group of the alkyl polyglycoside(s) contains 8 to 16 carbon atoms.

6. A hair shampoo composition according to any preceding claim wherein the alkyl group of the alkyl polyglycoside(s) contains 8 to 14 carbon atoms.

7. Use as a hair shampoo of a composition which comprises in addition to water:
(a) one or more alkyl polyglycoside(s) where the alkyl group contains 5 to 30 carbon atoms
(b) one or more acyl lactylate(s) of the following structure (1)
(R¹CO-(O-CHCH₃-CO)ₐ O)_{b} M
where R¹CO represents a C₆ to C₃₄ acyl radical: a is an integer of from 1 to 4; b is 1 or 2; and M represents H or a cosmetically-acceptable counterion of the valency 1 or 2;
wherein the weight ratio of acyl lactylate(s) to alkyl polyglycoside(s) is greater than or equal to 1:6.

## Patentansprüche

1. Haarshampoozusammensetzung, die zusätzlich zu Wasser
(a) ein oder mehrere Polyglycosid(e) mit 5 bis 30 Kohlenstoffatomen in der Alkylgruppe
(b) ein oder mehrere Acyllactylat(e) der folgenden Strukturformel (1)
(R¹CO-(O-CHCH₃-CO)ₐ O)_{b} M
worin R¹CO einen geradkettigen oder verzweigten C₆-C₃₄-Acylrest bedeutet, a eine ganze Zahl von 1 bis 4 ist, b 1 oder 2 ist und M H oder ein kosmetisch annehmbares Gegenion mit der Valenz 1 oder 2 bedeutet, enthält, wobei
das Gewichtsverhältnis von Acyllactylat(en) zu Alkylpolyglycosid(en) ≥ 1:6 ist.

2. Zusammensetzung nach Anspruch 1, worin das Gewichtsverhältnis von Acyllactylat(en) zu Alkylpolyglycosid(en) im Bereich von etwa 1:6 bis 2:1 liegt.

3. Haarshampoozusammensetzung nach Anspruch 1 oder Anspruch 2, worin R¹CO einen geradkettigen C₈-C₁₈-Acylrest bedeutet.

4. Haarshampoozusammensetzung nach einem der vorhergehenden Ansprüche, worin R¹CO einen geradkettigen C₁₀-C₁₄-Acylrest bedeutet.

5. Haarshampoozusammensetzung nach einem der vorhergehenden Ansprüche, worin die Alkylgruppe des Alkylpolyglycosids/der Alkylpolyglycoside 8 bis 16 Kohlenstoffatome enthält.

6. Haarshampoozusammensetzung nach einem der vorhergehenden Ansprüche, worin die Alkylgruppe des Alkylpolyglycosids/der Alkylpolyglycoside 8 bis 14 Kohlenstoffatome enthält.

7. Verwendung einer Zusammensetzung, die zusätzlich zu Wasser
(a) ein oder mehrere Polyglycosid(e) mit 5 bis 30 Kohlenstoffatomen in der Alkylgruppe
(b) ein oder mehrere Acyllactylat(e) der folgenden Strukturformel (1)
(R¹CO-(O-CHCH₃-CO)ₐ O)_{b} M
worin R¹CO einen geradkettigen oder verzweigten C₆-C₃₄-Acylrest bedeutet, a eine ganze Zahl von 1 bis 4 ist, b 1 oder 2 ist und M H oder ein kosmetisch annehmbares Gegenion mit der Valenz 1 oder 2 bedeutet, enthält,
wobei das Gewichtsverhältnis von Acyllactylat(en) zu Alkylpolyglycosid(en) ≥ 1:6 ist,
als Haarshampoo.

## Revendications

1. Composition de shampooing capillaire qui comprend, outre l'eau :
(a) un (ou plusieurs) alkylpolyglycoside(s) dans lequel le groupe alkyle contient 5 à 30 atomes de carbone ;
(b) un (ou plusieurs) lactylate (s) d'acyle de structure (1) suivante :
(R¹CO-(O-CHCH₃-CO)ₐO)_{b} M (1)
dans laquelle R¹CO représente un radical acyle en C₆₋₃₄; ramifié ou à chaîne droite ; a est un nombre entier de 1 à 4 ; b est 1 ou 2 ; et M représente H ou un contre-ion acceptable sur le plan cosmétique de valence 1 ou 2 ;
dans laquelle le rapport pondéral lactylate(s) d'acyle/alkylpolyglycoside(s) est supérieur ou égal à 1:6.

2. Composition selon la revendication 1, dans laquelle le rapport pondéral lactylate(s) d'acyle/alkylpolyglycoside(s) est dans la gamme d'environ 1:6 à 2:1.

3. Composition de shampooing capillaire selon la revendication 1 ou 2, dans laquelle R¹CO représente un radical acyle à chaîne droite en C₈₋₁₈.

4. Composition de shampooing capillaire selon l'une quelconque des revendications précédentes, dans laquelle R¹CO représente un radical acyle à chaîne droite en C₁₀₋₁₄.

5. Composition de shampooing capillaire selon l'une quelconque des revendications précédentes, dans laquelle le groupe alkyle de l'alkylpolyglycoside (des alkylpolyglycosides) contient 8 à 16 atomes de carbone.

6. Composition de shampooing capillaire selon l'une quelconque des revendications précédentes, dans laquelle le groupe alkyle de l'alkylpolyglycoside (des alkylpolyglycosides) contient 8 à 14 atomes de carbone.

7. Utilisation comme shampooing capillaire d'une composition qui comprend , outre l'eau :
(a) un (ou plusieurs) alkylpolyglycoside(s) dans lequel le groupe alkyle contient 5 à 30 atomes de carbone ;
(b) un (ou plusieurs) lactylate d'acyle(s) de structure (1) suivante :
(R¹CO-(O-CHCH₃-CO)ₐO)_{b} M (1)
dans laquelle R¹CO représente un radical acyle en C₆₋₃₄, ; a est un nombre entier de 1 à 4, b est 1 ou 2 ; et M représente H ou un contre-ion acceptable sur le plan cosmétique de valence 1 ou 2 ; et
dans laquelle le rapport pondéral lactylate(s) d'acyle/alkylpolyglycoside(s) est supérieur ou égal à 1:6.
